# EUROPEAN PATENT SPECIFICATION

(11) **EP 4 561 523 B1**
(45) Date of publication and mention of the grant of the patent: **04.11.2026**
(21) Application number: 23749044.6
(22) Date of filing: 28.07.2023
(51) Int. Cl.: A61K 8/35, A61K 8/37, A61Q 17/04

(54) **COMPOSITION COMPRISING A PHOTOCHEMICAL ULTRAVIOLET ABSORBER PRECURSOR AND A COMPOUND FOR INCREASING THEIR PHOTOCHEMICAL CONVERSION RATE**
ZUSAMMENSETZUNG MIT EINEM PHOTOCHEMISCHEN UV-ABSORBERVORLÄUFER UND EINER VERBINDUNG ZUR ERHÖHUNG DER PHOTOCHEMISCHEN UMWANDLUNGSRATE
COMPOSITION COMPRENANT UN PRÉCURSEUR ABSORBEUR D'ULTRAVIOLETS PHOTOCHIMIQUE ET UN COMPOSÉ POUR AUGMENTER SA VITESSE DE CONVERSION PHOTOCHIMIQUE

(30) Priority: 29.07.2022 EP 22382742
(43) Date of publication of application: 04.06.2025
(73) Proprietor: Roka Furadada, SL, 08030 Barcelona (ES)
(72) Inventor: CAMARGO SANROMÀ, Judit, 08030 BARCELONA (ES); DELGADO GARCÍA, Irene, 08041 BARCELONA (ES); AYNETO CARRANZA, Laura, 08018 BARCELONA (ES)
(74) Representative: ZBM Patents - Zea, Barlocci & Markvardsen
(86) International application number: PCT/EP2023/070972
(87) International publication number: WO 2024/023286

(56) References cited:
- WO-A1-2016/206962
- WO-A1-2017/036662
- WO-A2-2005/009341
- WO-A2-2006/100225

## Description

This application claims the benefit of European Patent Application EP22382742.9 filed on July 29, 2022.

### Technical Field

The present invention relates to the field of cosmetics, in particular, it relates to a cosmetic composition comprising a photochemical precursor of ultraviolet (UV) absorber and a phenylethyl ester, and to a method to photochemically convert photochemical UV absorber precursors into the UV absorber at a high conversion rate.

### Background Art

Overexposure to the sun's invisible rays, ultraviolet A (UVA, 320-400 nm) and ultraviolet B (UVB, 290-320 nm) can cause skin damage. The damage can be immediate and long-term, with effects ranging from sunburn, rashes, and cell and tissue damage to premature wrinkling and skin cancer.

Sunscreens help to prevent sunburn and reduce the harmful effects of the sun such as premature skin aging and skin cancer. Sunscreens should be applied between 20 and 30 minutes before sun exposure.

It is known that excessive exposure to both UVB radiation (290-320 nm) and UVA radiation (320-400 nm) has a damaging effect for skin. UVB is the most erythemogenic solar radiation reaching the surface of the earth. Sun Protection Factor (SPF) indicates the degree of protection against UVB induced erythema. The SPF represents the length of time that sunscreen-protected skin can be exposed to UV rays before a minimal redness (erythema) appears, compared to the length of time it takes on unprotected skin. Therefore, SPF denotes principally the degree of protection against UVB. Higher SPF sunscreen products have led to the use of multiple individual sunscreen active agents used in combinations at maximum concentrations that may interact.

Avobenzone (4-*tert*-butyl-4'-methoxydibenzoylmethane, Parsol 1789) is one of the most commonly used UVA filters since it provides superior protection through a large portion of the UVA range. However, concerns have been raised regarding its photostability, as well as of other dibenzoylmethane derivatives, and its potential to degrade other sunscreen ingredients in products in which it is used.

Several benzoic acid esters have been described as photochemical UV absorber precursors. Their photoprotective activity is due to the fact that they are susceptible to be photochemically converted *in situ* to a sunscreen active agent with enhanced UV protection ability.

As an example, the avobenzone vinyl ester precursor, methoxyphenylethenyl t-butylbenzoate (also known as 1-(4-methoxyphenyl)-vinyl 4-tert-butylbenzoate or 1-(4-methoxyphenyl)ethenyl 4-tert-butylbenzoate, CAS 910910-36-0), was firstly proposed as a solution to overcome the above mentioned stability problems.

Methoxyphenylethenyl t-butylbenzoate converts to avobenzone according to the following reaction:

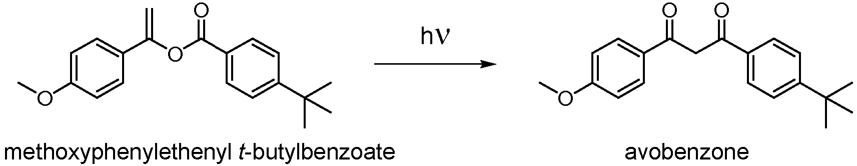

However, conversion into avobenzone proceeds relatively slowly and incompletely, what has the drawback that a delay in the action of avobenzone as an UVA filter is produced.

WO2016206962 A2 discloses a composition comprising a dialkyl adipate and an avobenzone precursor, which aims to overcome the slow conversion of avobenzone precursors into avobenzone.

Therefore, there is still a need of providing a sunscreen composition containing a photochemical UV absorber precursors such as methoxyphenylethenyl t-butylbenzoate that overcomes the problems of the prior art formulations containing such a precursor.

### Summary of Invention

The present inventors, in an attempt for developing an improved sunscreen formulation comprising certain benzoic acid esters which are photochemical UV absorber precursors, such as such as methoxyphenylethenyl t-butylbenzoate (which is photochemically converted to avobenzone), have found that by combining the mentioned benzoic acid esters with a phenylethyl ester, the photochemical conversion of the benzoic acid esters to their corresponding UV absorber takes place at a higher rate than in the absence of the mentioned phenylethyl ester.

Surprisingly, it has been found that the incorporation of a phenylethyl ester such as phenethyl benzoate (also known as 2-phenylethyl benzoate) in a composition comprising certain benzoic acid esters, such as methoxyphenylethenyl t-butylbenzoate, and more particularly in a sunscreen formulation comprising some UV sunscreen active agents, allows obtaining a stable sunscreen composition providing a quick and high protection against both UVA and UVB radiation. Besides, the combination of the phenylethyl ester with the mentioned benzoic acid esters allows increasing the photostability of the composition, in particular of the sunscreen formulation comprising the UV sunscreen active agents. As it can be seen in Tables 2 to 8, with phenethyl benzoate (PEB), dibutyl adipate (DBA), and diisopropyl adipate (DIPA) the absorbance values are maintained up to 10 MED, while with the rest of the emollients the absorbance values decrease as the radiation increases from approximately 2.5 MED.

Thus, an aspect the present invention refers to a composition comprising:
- an effective amount of a compound of formula (I), a pharmaceutically or a cosmetically acceptable salt thereof, or a stereoisomer of any of them or mixtures thereof, 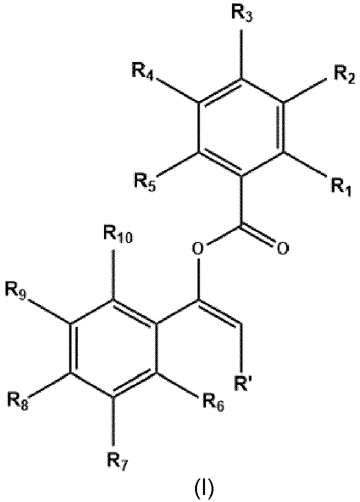 wherein: R' is selected from the group consisting of H, (C₁-C₆)-alkyl, and (C₃-C₆)-cycloalkyl; R₁, R₂, R₄, R₅, R₆, R₇, R₉, and R₁₀ are a radical independently selected from the group consisting of H, hydroxy, amino, (C₁-C₆)-alkyl, (C₁-C₆)-alkoxy, (C₁-C₆)-alkylamino, and (C₁-C₆)-dialkylamino; and R₃,and R₈ are independently selected from the group consisting of (C₁-C₆)-alkyl, (C₁-C₆)-alkoxy, hydroxy, amino, (C₁-C₆)-alkylamino, and (C₁-C₆)-dialkylamino; such as 1-(4-methoxyphenyl)ethenyl 4*-tert-*butylbenzoate; and
- an effective amount of a phenylethyl ester selected from the group consisting of phenethyl benzoate, 2-phenylethyl toluate, di-2-phenylethyl phthalate, and 1-phenylethyl benzoate;
together with one or more pharmaceutically or cosmetically acceptable excipients or carriers.

Another aspect of the present invention refers to the use of the composition as defined herein above and below to provide protection for the skin or hair against the sun's rays or other sources of UV radiation. This aspect can also be formulated as a composition as defined herein above and below for use to provide protection for the skin or hair against the sun's rays or other sources of UV radiation. This use is related to a method for protecting a human or animal living body from UV radiation comprising treating said human or animal living body with an effective amount of the composition as defined herein above and below. Particularly, the human or animal living body is a human being.

In another aspect, the invention provides a method for increasing the photochemical conversion rate of a compound of formula (I) as defined above or below into a compound of formula (II), a pharmaceutically or a cosmetically acceptable salt thereof, or a stereoisomer of any of them or mixtures thereof, 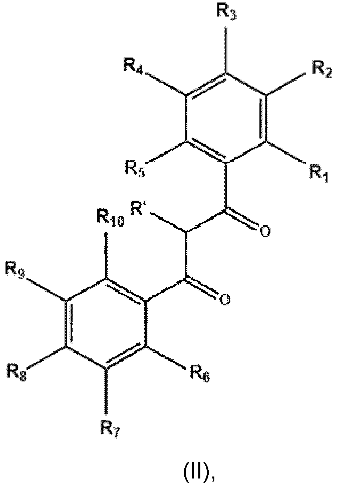 wherein: R' is selected from the group consisting of H, (C₁-C₆)-alkyl, and (C₃-C₆)-cycloalkyl; R₁, R₂, R₄, R₅, R₆, R₇, R₉, and R₁₀ are a radical independently selected from the group consisting of H, hydroxy, amino, (C₁-C₆)-alkyl, (C₁-C₆)-alkoxy, (C₁-C₆)-alkylamino, and (C₁-C₆)-dialkylamino; and R₃,and R₈ are independently selected from the group consisting of (C₁-C₆)-alkyl, (C₁-C₆)-alkoxy, hydroxy, amino, (C₁-C₆)-alkylamino, and (C₁-C₆)-dialkylamino, in a composition, the method comprising adding to the composition an effective amount of a phenylethyl ester selected from the group consisting of phenethyl benzoate, 2-phenylethyl toluate, di-2-phenylethyl phthalate, and 1-phenylethyl benzoate. This aspect can also be formulated as the use of a phenylethyl ester selected from the group consisting of phenethyl benzoate, 2-phenylethyl toluate, di-2-phenylethyl phthalate, and 1-phenylethyl benzoate for increasing the photochemical conversion rate of a compound of formula (I) as defined herein above and below into a compound of Formula (II) , a pharmaceutically or a cosmetically acceptable salt thereof, or a stereoisomer of any of them or mixtures thereof, as defined herein above and below in a cosmetic composition.

An example of compound of formula (I) is methoxyphenylethenyl t-butylbenzoate, which is photochemically converted to avobenzone.

### Brief Description of Drawings

Fig. 1 shows the results of the UVA-PF values for the photoactivation study with a solution of 2 % (w/w) methoxyphenylethenyl t-butylbenzoate in different emollients: phenethyl benzoate (PEB), dibutyl adipate (DBA), diisopropyl adipate (DIPA), C12-15 alkyl benzoate (C12-15 AB), dicaprylyl carbonate (DCC), cocoglycerides (CCG), and caprylic/capric triglycerides (CCT).
Fig. 2 shows the results of the SPF values for the photoactivation study with a solution of 2 % (w/w) methoxyphenylethenyl t-butylbenzoate in different emollients.
Fig. 3 shows the results of the absorbance at 360 nm (Abs 360 nm) for the photoactivation study with a solution of 2 % (w/w) methoxyphenylethenyl t-butylbenzoate in different emollients.
Fig. 4 shows the results of the absorbance spectrum of a solution of methoxyphenylethenyl t-butylbenzoate 2 wt.% in phenethyl benzoate at different irradiation doses.
Fig. 5 shows the results of the absorbance at 360 nm (Abs 360 nm) of a solution of methoxyphenylethenyl t-butylbenzoate 2 wt.% in phenethyl benzoate, C12-15 alkyl benzoate, or dibutyl adipate at different irradiation doses.
Fig. 6-a, 6-b, and 6-c show the activation speed rate of methoxyphenylethenyl t-butylbenzoate (PvB) in phenethyl benzoate (PEB), dibutyl adipate (DBA), and C12-15 alkyl benzoate (C12-15 AB), respectively.
Fig. 7 shows the results of the UVA-PF values obtained with several oil phases containing: A: a mixture of homosalate, bis-ethylhexyloxyphenol methoxyphenyl triazine, diethylamino hydroxybenzoyl hexyl benzoate, ethylhexyl triazone, and dibutyl adipate (Comparative Example 7); B: the mixture of A plus methoxyphenylethenyl t-butylbenzoate (Comparative Example 8); C: the mixture of A plus phenethyl benzoate (Comparative Example 9); and D: the mixture of A and methoxyphenylethenyl t-butylbenzoate and phenethyl benzoate (Example 2).
Fig. 8 shows the results of the absorbance at 360 nm (Abs 360 nm) obtained with the oil phases A, B, C, D mentioned above.
Fig. 9 shows the results of the SPF values obtained with the oil phases A, B, C, D mentioned above.

### Detailed description of the invention

All terms as used herein in this application, unless otherwise stated, shall be understood in their ordinary meaning as known in the art. Other more specific definitions for certain terms as used in the present application are as set forth below and are intended to apply uniformly through-out the specification and claims unless an otherwise expressly set out definition provides a broader definition. For the purposes of the present disclosure, any ranges given include both the lower and the upper end-points of the range. Ranges given, such as temperatures and amounts, should be considered approximate, unless specifically stated.

The term "sunscreen active agent", as used herein, relate to materials, singly or in combination, that are regarded as acceptable for use as active sun screening ingredients relative to their ability to absorb, scatter, or reflect UV radiation. Such compounds are generally described as being UVA, UVB, or UVA/UVB sunscreen active agents, depending on the wavelength range of the UV radiation.

The term "UV-A" refers to ultraviolet electromagnetic radiation with a wavelength from about 320 nm to about 400 nm.

The term "UV-B" refers to ultraviolet electromagnetic radiation with a wavelength from about 290 nm to about 320 nm.

The term "sunscreen composition" refers to a composition intended for topical application to provide protection for the skin or hair against the sun's rays or other sources of UV radiation.

The term "UVA absorber" or "UVB absorber" refers to active agents with the ability to absorb UVA or UVB radiations, respectively.

The Sun Protection Factor (SPF) refers to the ratio of minimum erythemal dose (MED) on skin protected by a sunscreen product to the minimal erythemal dose on the same unprotected skin.

The minimal erythema dose (MED) is the amount of UV radiation that will produce minimal erythema (sunburn or redness caused by engorgement of capillaries) of an individual's skin within a few hours following exposure.

The UVA protection factor (UVA-PF) means the ratio of the minimum UVA radiation dose necessary to induce persistent pigment darkening on the skin protected by a sunscreen product to the minimal UVA radiation dose necessary to induce the minimal darkening effect on the same unprotected skin.

The term "effective amount" as used herein refer to a sufficient amount of the active ingredient specified to provide the sought effect or the desired performance attributes.

All percentages as used herein are by weight of the total composition, unless otherwise stated. In all the compositions of the present disclosure, the sum of all components is 100%.

As used herein, the indefinite articles "a" and "an" are synonymous with "at least one" or "one or more." Unless indicated otherwise, definite articles used herein, such as "the", also include the plural of the noun.

Throughout the description and claims, the terms (C₁-C₆)-alkyl, (C₁-C₆)-alkoxy, (C₁-C₆)-alkylamino, and (C₁-C₆)-dialkylamino shall be construed as straight or branched.

As mentioned above, an aspect of the invention relates to a composition, for topical use, comprising a compound of formula (I), a pharmaceutically or a cosmetically acceptable salt thereof, or a stereoisomer of any of them, or mixtures thereof, wherein: R' is selected from the group consisting of H, (C₁-C₆)-alkyl, and (C₃-C₆)-cycloalkyl; R₁, R₂, R₄, R₅, R₆, R₇, R₉, and R₁₀ are a radical independently selected from the group consisting of H, hydroxy, amino, (C₁-C₆)-alkyl, (C₁-C₆)-alkoxy, (C₁-C₆)-alkylamino, and (C₁-C₆)-dialkylamino; and R₃,and R₈ are independently selected from the group consisting of (C₁-C₆)-alkyl, (C₁-C₆)-alkoxy, hydroxy, amino, (C₁-C₆)-alkylamino, and (C₁-C₆)-dialkylamino; and a phenylethyl ester selected from the group consisting of phenethyl benzoate, 2-phenylethyl toluate, di-2-phenylethyl phthalate, and 1-phenylethyl benzoate; together with one or more pharmaceutically or cosmetically acceptable excipients or carriers.

It is understood that the composition is a pharmaceutical or a cosmetic composition.

Compounds of formula (I) when R' is different to H exhibit cis-trans isomerism. Thus, the compound of formula (I) can consist of a mixture of isomers. When desired, the isomers can be separated by conventional means of purification.

In a particular embodiment, in the compound of formula (I) and in compound of formula (II), R' is H.

In another particular embodiment, in the compound of formula (I) and in compound of formula (II) R₁, R₂, R₄, R₅, R₆, R₇, R₉, and R₁₀ are a radical independently selected from the group consisting of H, hydroxy, amino, and methyl.

In another particular embodiment, in compound of formula (I) and in compound of (II), R₂, R₄, R₇, and R₉ are H.

In another particular embodiment, in compound of formula (I) and in compound of formula (II) R', R₁, R₂, R₄, R₅, R₆, R₇, R₉, and R₁₀ are H; R₃ is tert-butyl and R₈ is methoxyl. The preferred compounds of formula (I) are the following: 1-phenylvinyl 4-methoxybenzoate; methoxyphenylethenyl t-butylbenzoate; 1-(4-tert-butylphenyl) vinyl 4-methoxybenzoate; and 1-phenylvinyl 4-tert-butylbenzoate. More preferably, the compound of formula (I) is methoxyphenylethenyl t-butylbenzoate.

The term "cosmetically acceptable" refers to that excipients or carriers suitable for use in contact with human skin without undue toxicity, incompatibility, instability, allergic response, among others.

The expression "pharmaceutically acceptable excipients or carriers" refers to pharmaceutically acceptable materials, compositions or vehicles. Each component must be pharmaceutically acceptable in the sense of being compatible with the other ingredients of the pharmaceutical composition. It must also be suitable for use in contact with the tissue or organ of humans and animals without excessive toxicity, irritation, allergic response, immunogenicity or other problems or complications commensurate with a reasonable benefit/risk ratio.

The term "pharmaceutically or cosmetically acceptable salt" refers to a non-toxic salt. As some of the compounds of formula (I) are basic compounds, salts may be prepared with pharmaceutically or cosmetically acceptable non-toxic acids. Compound of formula (II) may also be in the form of a pharmaceutically or a cosmetically acceptable salt. The term "pharmaceutically or cosmetically acceptable salt" used herein for any of compounds (I) and (II) encompasses any salt formed from organic and inorganic acids, such as hydrobromic, hydrochloric, phosphoric, nitric, sulfuric, acetic, adipic, aspartic, benzenesulfonic, benzoic, citric, ethanesulfonic, formic, fumaric, glutamic, lactic, maleic, malic, malonic, mandelic, methanesulfonic, 1,5-naphthalendisulfonic, oxalic, pivalic, propionic, p- toluenesulfonic, succinic, tartaric acids, and the like.

Compounds of formula (I) can be obtained by a great variety of methods disclosed in the art. Thus, WO2006/100225A1 discloses benzoic acid ester compounds and processes for their preparation based on the reaction of an acyl halide with an intermediate silyl enol ether according to the following scheme, in which R₁-R₂₁ and R' have the values defined therein.

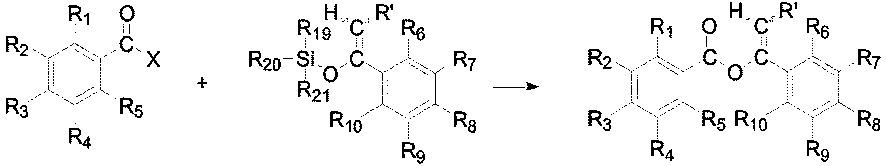

The preparation of pharmaceutically or cosmetically acceptable salts of the compound of formula (I) can be carried out by methods known in the art. For instance, they can be prepared from the parent compound, which contains a basic moiety, by conventional chemical methods. Generally, such salts are, for example, prepared by reacting the free base form of these compounds with a stoichiometric amount of the appropriate pharmaceutically or cosmetically acceptable acid in water or in an organic solvent or in a mixture of them.

The compounds of formula (I) may be in crystalline form either as free solvation compounds or as solvates (e.g., hydrates) and it is intended that both forms are within the scope of the present invention. Methods of solvation are generally known within the art.

The term "solvate" refers to a molecular complex comprising the compound of formula (I) or a salt thereof, and a stoichiometric or non-stoichiometric amount of one or more solvent molecules bound by non-covalent intermolecular forces. When the one or more solvent molecules forming part of the molecular complex is water, the solvate is a hydrate.

Compound of formula (I) or its salts can be further purified for instance by crystallization in an appropriate solvent. Examples of appropriate solvent are (C₂-C₆)-alcohol such as ethanol, or 2-propanol. Compound of formula (I) can be obtained with a purity equal to or higher than 99% by HPLC. In a particular embodiment, compound of formula (I) is obtained with a purity equal to or higher than 99.5%. Other appropriate solvent can for the purification step can be (C₆-C₈)-aromatic hydrocarbons such as toluene or xylene, (C₁-C₃)-chlorine containing solvents such as dichloromethane, dichloroethane, or mixtures of (C₆-C₈)-aromatic hydrocarbons and (C₂-C₆)-alcohols. In a particular embodiment, the solvent mixture is 2-propanol/toluene.

As mentioned above, the composition of the invention also comprises a phenylethyl ester. Although usually used as emollients, in the composition of the present invention the phenylethyl esters are used for increasing the conversion rate of the photochemical UV absorber precursors compared to the conversion in the absence of the mentioned phenylethyl ester.

In an embodiment, optionally in combination with one or more features of the embodiments defined above, the phenylethyl ester is phenethyl benzoate.

In another embodiment of the composition of the present disclosure, optionally in combination with one or more features of the embodiments defined above, the compound of formula (I) is in an amount from 0.1 wt.% to 6 wt.%, preferably from 0.5 wt.% to 5 wt.%, relative to the total weight of the composition.

In another embodiment of the composition of the present disclosure, optionally in combination with one or more features of the embodiments defined above, the phenylethyl ester is in an amount from 0.1 wt.% to 40 wt.%, preferably from 0.5 wt.% to 25 wt.%, more preferably from 2 wt.% to 15 wt.%, even more preferably from 2 wt.% to 6 wt.%, relative to the total weight of the composition.

In another embodiment of the composition of the present disclosure, optionally in combination with one or more features of the embodiments defined above, the compound of formula (I) is in an amount from 0.1 wt.% to 6 wt.%, preferably from 0.5 wt.% to 5 wt.%; and the phenylethyl ester is in an amount from 0.1 wt.% to 40 wt.%, preferably from 0.5 wt.% to 25 wt.%, more preferably from 2 wt.% to 15 wt.%, even more preferably from 2 wt.% to 6 wt.%, relative to the total weight of the composition.

In another embodiment, optionally in combination with one or more features of the embodiments defined above, the composition further comprises a UV sunscreen active agent. Particularly, the composition of the invention can be a sunscreen composition.

As an example, the UV sunscreen active agent can be in an amount such that the composition has an SPF of at least 30, preferably from at least 35 to at least 50, more preferably of at least 50. The amount of UVB sunscreen active agent will depend on the specific UVB sunscreen active agent or mixture of UVB, UVA, or UVA/UVB sunscreen active agents used, and can readily be determined by those skilled in the art.

Examples of UV sunscreen active agents include: octyl salicylate (2-ethylhexyl salicylate, Escalol 587); pentyl dimethyl PABA; octyl dimethyl PABA (padimate O, Escalol 507); benzophenone-1 ; benzophenone-6 (Uvinul D-49); 2-(2H-benzotriazole-2-yl)-4,6-di-tert-pentylphenol (Uvinul 3028); ethyl-2-cyano-3,3-diphenylacrylate (Uvinul 3035); homomenthyl salicylate (homosalate); bis-ethylhexyloxyphenol methoxyphenyl triazine (bemotrizinol, Escalol S); methyl-(1,2,2,6,6-pentamethyl-4-piperidyl)-sebacate (Uvinul 4092H); benzenepropanoic acid, 3,5-bis (1,1-dimethyl-ethyl)-4-hydroxy-, C7-C9 branched alkyl esters (Irganox 1135); 2-(2H-benzotriazole-2-yl)-4-methylphenol (Uvinul 3033P); diethylhexyl butamido triazone (iscotrizinol, Uvasorb HEB); amyl dimethyl PABA (lisadimate, glyceryl PABA); 4,6- bis(octylthiomethyl)-o-cresol (Irganox 1520); CAS number 65447-77-0 (Uvinul 5062H, Uvinul 5062GR); red petroleum; ethylhexyl triazone (Uvinul T-150); octocrylene (Escalol 597); isoamyl-p-methoxycinnamate (amiloxate, Neo Heliopan E1000); drometrizole; titanium dioxide (including as nanomaterial); 2,4-di-tert-butyl-6-(5-chloro-2H-benzotriazole-2-yl)-phenol (Uvinul 3027); 2-hydroxy-4-octyloxybenzophenone (Uvinul 3008); benzophenone-2 (Uvinul D-50); diisopropyl methylcinnamate; PEG-25 PABA; 2-(1,1-dimethylethyl)-6-[[3-(1,1-demethylethyl)-2-hydroxy-5-methylphenyl]methyl-4-methylphenyl acrylate (Irganox 3052); drometrizole trisiloxane (Mexoryl XL); menthyl anthranilate (meradimate); bis-(1,2,2,6,6-pentamethyl-4-piperidyl)-sebacate; butyl methoxydibenzoylmethane (avobenzone, Escalol 517); 2-ethoxyethyl p-methoxycinnamate (cinnoxate); benzylidene camphor sulfonic acid (Mexoryl SL); dimethoxyphenyl-[1-(3,4)]-4,4-dimethyl-1,3-pentanedione; zinc oxide (including as nanomaterial); N,N'-hexane-1,6-diyl-bis [3-(3,5-di-tert-butyl-4-hydroxyphenylpropionamide)] (Irganox 1098); pentaerythritol tetrakis[3-(3,5-di-tert-butyl-4-hydroxyphenyl)propionate] (Irganox 1010); 2,6-di-tert-butyl-4-[4,6-bis(octylthio)-1,3,5-triaziN-2-ylamino] phenol (Irganox 565); 2-(2H-benzotriazole-2-yl)-4,6-bis(1-methyl-1-phenylethyl)phenol (Uvinul 3034); trolamine salicylate (triethanolamine salicylate); diethylanolamine p-methoxycinnamate (DEA methoxycinnamate); polysilicone-15 (Parsol SLX); CAS number 152261-33-1 (Uvinul 5050H); 4-methylbenzylidene camphor (Eusolex 6300, Parsol 5000); bisoctrizole (Tinosorb M), including as nanomaterial; benzenamine, N-phenyl-, reaction products with 2,4,4-trimethylpentene (Irganox 50507); sulisobenzone, Escalol 577); (2-ethylhexyl)-2-cyano-3,3-diphenylacrylate (Uvinul 3039); digalloyl trioleate; polyacrylamido methylbenzylidene camphor; glyceryl ethylhexanoate dimethoxycinnamate; 1,3-bis-[(2'-cyano-3',3'- diphenylacryloyl)oxy]-2,2-bis-{[(2'-cyano-; bis-(2,2,6,6-tetramethyl-4-piperidyl)-sebacate (Uvinul 4077H); benzophenone-5 (sulisobenzone sodium); 1,3,5-tris(3,5-di-tert-butyl-4- hydroxybenzyl)-1,3,5-triazine-2,4,6(IH,3H,5H)-trione (Irganox 3114); 1,3,5-Triazine, 2,4,6-tris(1,1'-biphenyl)-4-yl-, including as nanomaterial; hexamethylendiamine (Uvinul 4050H); benzophenone-8 (dioxybenzone); ethyl-4- bis(hydroxypropyl) aminobenzoate (roxadimate); 6-tert-butyl-2-(5-chloro-2H-benzotriazole- 2-yl)-4-methylphenol (Uvinul 3026); p-aminobenzoic acid (PABA); 3,3',3",5,5',5"-hexa-tert-butyl-α-α'-α"-(mesitylene-2,4,6-triyl)tri-p-cresol (Irganox 1130); lawsone with dihydroxyacetone; benzophenone-9 (Uvinul DS-49); benzophenone-4 (sulisobenzone); ethylhexyl dimethoxy benzylidene dioxoimidazoline propionate; N,N'-bisformyl-N,N'-bis- (2,2,6,6-tetramethyl-4-piperidinyl)-; 3-benzylidene camphor (Mexoryl SD); terephthalylidene dicamphor sulfonic acid; camphor benzalkonium methosulfate (Mexoryl SO); bisdisulizole disodium (Neo Heliopan AP); etocrylene; ferulic acid; 2-(2H-benzotriazole-2-yl)-4-(1,1,3,3-tetramethylbutyl)-phenol (Uvinul 3029); ecamsule (Mexoryl SX); 4,6-to(dodecylthiomethyl)-o-cresol (Irganox 1726); beta-2-glucopyranoxy propyl hydroxy benzophenone; phenylbenzimidazole sulfonic acid (ensulizole, Eusolex 232, Parsol HS); benzophenone-3 (oxybenzone, Escalol 567); diethylamine hydroxybenzoyl hexylbenzoate (Uvinul A Plus); 3',3'-diphenylacryloyl)oxy]methyl}-propane (Uvinul 3030); 3,3#-(1,4-phenylene)bis(5,6-diphenyl-1,2,4-triazine); 2-ethoxyethyl (2Z)-2-cyano-2-[3-(3-methoxypropylamino) cyclohex-2-en-1-ylidene]acetate and ethylhexyl p-methoxycinnamate (Escalol 557).

The UV sunscreen active agent can be a UVB sunscreen active agent, a UVA sunscreen active agent, a UVA/UVB sunscreen active agent, or a mixture thereof. It is generally desirable to formulate sunscreen compositions with more than one UV sunscreen active agent, particularly to extend the range of UV radiation absorption provided, more particularly, to cover a broad protection against both UVA and UVB radiation.

In another embodiment, optionally in combination with one or more features of the embodiments defined above, the UV sunscreen active agent is in an amount from 0.1 wt.% to 50 wt.%, preferably from 0.5 wt.% to 45 wt.%, more preferably from 1.0 wt.% to 35 wt.% relative to the total weight of the composition.

The amount of UV sunscreen active agent will depend on the specific UV sunscreen active agent or mixture of UV sunscreen active agents and can readily be determined by those skilled in the art. A list of UV sunscreen active agents allowed in cosmetic products can be found in Annex VI to Regulation (EC) No 1223/2009 related to cosmetic products, within the EU's official database for cosmetic ingredients (CosIng), wherein it is indicated their maximum concentration in ready for use preparations.

In an embodiment, optionally in combination with one or more features of the embodiments defined above, the UV sunscreen active agent comprises 3,3,5-trimethylcyclohexyl 2-hydroxybenzoate (homosalate), ethylhexyl triazone, diethylamino hydroxybenzoyl hexyl benzoate, and bis-ethylhexyloxyphenol methoxyphenyl triazine.

In another embodiment, 3,3,5-trimethylcyclohexyl 2-hydroxybenzoate (homosalate) is in an amount from 2 wt.% to 10 wt.%, ethylhexyl triazone is in an amount from 0.5 wt.% to 5 wt.%, diethylamino hydroxybenzoyl hexyl benzoate is in an amount from 0.5 wt.% to 10 wt.% and bis-ethylhexyloxyphenol methoxyphenyl triazine is in an amount from 0.5 wt.% to 10 wt.% relative to the total weight of the composition.

In another embodiment, optionally in combination with one or more features of the embodiments defined above, the composition also comprises an emollient other than the phenylethyl ester.

Examples of appropriate emollients include, without being limited to, alkyl adipates, C12-15 alkyl benzoate, dicaprylyl carbonate, cocoglycerides, caprylic/capric triglicerides, propylene glycol dicaprylate/dicaprate, butylene glycol dicaprylate/dicaprate, coco-caprylate, coco-caprylate/caprate, butyloctyl salicylate, diisopropyl sebacate, isopropyl lauroyl sarcosinate, isononyl isononanoate, diethylhexyl 2,6-naphthalate, ricinus communis (castor) seed oil, prunus amygdalus dulcis oil, cocos nucifera (coconut) oil, neopentyl glycol diheptanoate, isoamyl laurate, squalane, isododecane, propylene glycol dibenzoate, C13-14 alkane, octyldodecyl neopentanoate, argania spinosa kernel oil, ethylhexyl palmitate, ethylhexyl stearate, dipropylhptyl carbonate, hexyl laurate, dicaprylyl ether, caprylyl caprylate/caprate, propylheptyl caprylate, cetearyl isononanoate, cetearyl ethylhexanoate, PEG-7 glyceryl cocoate, myristyl lactate, decyl oleate, undecane, tridecane, decyl oleate, cetyl palmitate, octyldodecanol, hexyldecanol, myristyl myristate, isopropyl myristate, isopropyl palmitate, hydrogenated polyisobutene, isohexadecane, isopropyl isostearate, isostearyl isostearate, isotidecyl isononanoate, diisopropyl dimer dilinoleate, dimethyl capramide, diisostearyl dimer dilinoleate, pentaerythrityl tetraisostearate,triisononanoin, triethylhexanoin, and triisostearin.. Preferably, the further emollient is a dialkyl adipates. Examples of dialkyl adipates include dimethyl adipate, diethyl adipate, dipropyl adipate, diisopropyl adipate, diisobutyl adipate, di-n-butyl adipate, di-2-ethylhexyl adipate and dicyclohexyl adipate. Preferably, the dialkyl adipate is dibutyl adipate or diisopropyl adipate, more preferably dibutyl adipate.

In an embodiment, optionally in combination with one or more features of the embodiments defined above, the emollient other than the phenylethyl ester is in an amount from 0.5 wt.% to 35 wt.%, preferably from 5 wt.% to 25 wt.%, more preferably from 5 wt.% to 20 wt.%, relative to the total amount of the composition, being the sum of all components 100%.

In another embodiment, optionally in combination with one or more features of the embodiments defined above, the UV sunscreen agent comprises an UVB sunscreen active agent, an UVA sunscreen active agent, and a UVA/UVB sunscreen active agent. In a particular embodiment, the compound of formula (I) is methoxyphenylethenyl t-butylbenzoate, and
- the methoxyphenylethenyl t-butylbenzoate is in an amount from 0.1 wt.% to 6 wt.%;
- the phenylethyl ester is in an amount from 0.1 wt.% to 40 wt.%;
- the UVB sunscreen active agent is in an amount from 0.1 wt.% to 25 wt.%;
- the UVA sunscreen active agent is in an amount from 0.1 wt.% to 15 wt.%;
- the UVA/UVB sunscreen active agent is in an amount from 0.1 wt.% to 15 wt.%; and
- the dialkyl adipate is in an amount from 0.5 wt.% to 35 wt.%
wherein the weight percentages are relative to the total weight of composition, being the sum of all components 100%.

In another particular embodiment,
- methoxyphenylethenyl t-butylbenzoate is in an amount from 0.5 wt.% to 5 wt.%;
- the phenylethyl ester is in an amount from 2 wt.% to 25 wt.%;
- the UVB sunscreen active agent is in an amount from 1 wt.% to 20 wt.%;
- the UVA sunscreen active agent is in an amount from 1 wt.% to 10 wt.%;
- the UVA/UVB sunscreen active agent is in an amount from 1 wt.% to 10 wt.%; and
- the dialkyl adipate is in an amount from 5 wt.% to 30 wt.%,
wherein the weight percentages are relative to the total weight of composition, being the sum of all components 100%.

The composition defined above can comprise other pharmaceutically or cosmetically acceptable excipients or carriers suitable for topical administration. Examples of these excipients or carriers include, without being limited to, dispersing agents, preservatives, oils, waxes, propellants, emulsifiers, surfactants, thickeners, humectants, pH-regulating agents, film-forming agents, chelating agents, vehicles, and mixtures thereof. The suitable cosmetically excipients or carriers and their amounts can vary depending on the specific formulation type and can be readily determined by those skilled in the art.

The excipients or carriers referred to above and discussed more particularly below are generally used in an amount from about 0.1 wt % to about 10 wt % of the compositon, unless otherwise stated.

Examples of suitable dispersing agents include those useful for dispersing organic or inorganic sunscreen agents in either a water phase, oil phase, or part of an emulsion, including, for example, chitosan.

Examples of suitable preservatives or preservative enhancers include phenoxyethanol, ethylhexylglycerol, alkanediols (e.g. 1,2-pentanediol, 1,2-hexanediol, 1,2-octanediol, 1,2-decanediol, 2-methyl-1,3-propanediol), hydroxyacetophenone, sodium levulinate, glyceryl caprylate, glyceryl undecylenate, p-anisic acid, o-cymen-5-ol, phenylpropanol, methylparaben, ethylparaben, propylparaben, potassium sorbate and sodium benzoate. The amount of the preservatives in the compositions of the present invention can be from 0.1 wt.% to 3 wt.%, relative to the total weight of the composition.

Examples of suitable antioxidants include, without being limited to, tocopheryl acetate, tocopherol, butylated hydroxytoluene (BHT), pentaerythrityl tetra-di-t-butyl hydroxyhydrocinnamate, diethylhexyl syringylidenemalonate, ascorbyl palmitate, and vitamin C, p-hydroxybenzoic acid and its esters, salicylates, cumarin derivatives, flavones, hydroxy or methoxy substituted benzophenones, uric or tannic acid and its derivatives, hydroquinone, and benzophenones. The amount of antioxidant in the compositions of the present invention can be from 0.001 wt.% to 1 wt.%, relative to the total weight of the composition.

Examples of oils include, without being limited to, animal oils, vegetable oils, mineral oils, and synthetic oils, particularly hydrogenated palm oil, hydrogenated castor oil, liquid paraffin, paraffin oil, purcellin oil, and silicone oils.

Examples of waxes include, without being limited to, animal waxes, fossil waxes, vegetable waxes, mineral waxes, and synthetic waxes, particularly beeswaxes, carnauba wax, candelilla wax, sugar cane wax, Japan wax, ozokerites, montan wax, microcrystalline waxes, and paraffin waxes.

Examples of suitable humectants include glycerin, ethoxylated glycerin, propoxylated glycerin, propylene glycol, dipropylene glycol, polypropylene glycol, polyethylene glycol, butylene glycol, pentylene glycol, propanediol, sorbitol, polypropylene glycol, polyethylene glycol,, L-arginine, sodium hyaluronate, hyaluronic acid, and mixtures thereof. The amount of humectant in the compositions of the present invention can be from 1 to 30 wt.%, preferably from 2 to 20 wt.% and optimally from about 5 to 10 wt.%, relative to the total weight of the composition.

Examples of suitable pH-regulating agents include acetic acid, lactic acid, citric acid, ethanolamine, formic acid, oxalic acid, potassium hydroxide, sodium hydroxide, triethanolamine, or mixtures thereof. The amount of the pH-regulating agent in the compositions of the present invention can be from 0.01 wt.% to 1 wt.%, relative to the total weight of the composition.

Examples of suitable film-forming agents include polyvinylpyrrolidone (PVP), copolymers of PVP such as PVP/hexadecene copolymer and PVP/eicosene copolymer, acrylates copolymers, acrylate/octylacrylamide copolymers, sodium polystyrene sulfonate, polyisobutene, polyacrylamide, polyvinyl alcohol,polyglycols, poly C10-30 ate, trimethylpentanediol/adipic acid/glycerin crosspolymer, pullulan, chitosan, polyurethane-62, polyester-7 and polyurethane-99. The amount of the film-forming agent in the compositions of the present invention can be from 0.01 wt.% to 10 wt.%.

Examples of suitable chelating agents include disodium EDTA, tetrasodium EDTA, trisodium EDTA, tetrasodium glutamate diacetate, disodium pyrophosphate, dipotassium pyrophosphate, phytic acid and sodium phytate. The amount of the chelating agent in the compositions of the present invention can be from 0.01 wt.% to 10 wt.%.

Examples of emulsifiers include amphoteric, anionic, cationic or nonionic emulsifiers. These emulsifiers are chosen appropriately according to the emulsion to be obtained (W/O, O/W, O/W/O or W/O/W). Examples of suitable emulsifiers include glyceryl stearate citrate, glyceryl stearate (self-emulsifying), stearic acid, stearate salts, lauryl glucoside, myristyl glucoside, polyglyceryl-6 laurate, potassium cetyl phosphate polyglyceryl 3-methylglycose distearate, polyglyceryl-2 diisostearate, behenyl alcohol, stearyl alcohol, PEG-100 stearate, polyglyceryl-6 Distearat, polyglyceryl-3 polyricinoleate, polyglycerin-3, cetyl alcohol, dimethicone copolyol phosphate, hexadecyl-D- glucoside, octadecyl-D-glucoside, sorbitan olivate, polyglyceryl-6 polyhydroxystearate, polyglyceryl-6 polyricinoleate, hydrogenated lecithin, lecithin, sodium cetearyl sulfate, sodium staroyl glutamate, polyglyceryl-10 stearate, cetearyl alcohol, cetearyl glucoside, glycol palmitate and cetearyl phosphate sulfate. The amount of emulsifier in the compositions of the present invention can be from 0.5 wt.% to 15 wt.%.

Examples of suitable surfactants include, without being limited to, non-ionic, ionic (either anionic or cationic), or zwitterionic (or amphoteric wherein the head of the surfactant contains two oppositely charged groups) surfactants. Examples of anionic surfactants include those based on sulfate, sulfonate or carboxylate anions such as perfluorooctanoate (PFOA or PFO), alkyl benzene sulfonate, soaps, fatty acid salts, or alkyl sulfate salts such as perfluorooctanesulfonate (PFOS), sodium dodecyl sulfate (SDS), ammonium lauryl sulfate, or sodium lauryl ether sulfate (SLES). Examples of cationic surfactants include those based on quaternary ammonium cations such as or alkyltrimethylammonium including cetyl trimethylammonium bromide (CTAB) a.k.a., or hexadecyl trimethyl ammonium bromide, cetylpyridinium chloride (CPC), polyethoxylated tallow amine (POEA), benzalkonium chloride (BAC), or benzethonium chloride (BZT). Examples of zwitterionic surfactants include dodecyl betaine, cocamidopropyl betaine, or coco ampho glycinate. Examples of non-ionic surfactants include alkyl poly(ethylene oxide), alkylphenol poly(ethylene oxide), copolymers of poly(ethylene oxide), poly(propylene oxide) (commercially called Poloxamers or Poloxamines), alkyl polyglucosides including octyl glucoside and decyl maltoside, fatty alcohols including cetyl alcohol and oleyl alcohol, cocamide MEA, cocamide DEA, or polysorbates including tween 20, tween 80, or dodecyl dimethylamine oxide. Preferably, the surfactant is foaming and skin friendly, including polysorbate 20 or 40, coco glucoside, lauryl glucoside, decyl glucoside, lauryl sulfates such as ammonium, sodium, magnesium, MEA, triethylamine (TEA), or mipa lauryl sulfate, cocamidopropyl betain, or sodium alkyl sulfosuccinates. The amount of the surfactant in the compositions of the present invention can be from 0.5 wt.% to 20 wt.%.

Examples of suitable thickeners include ethylcellulose, hydroxypropylcellulose, hydroxyethylcellulose, hydroxypropylmethylcellulose, polyethylene glycol, microcrystalline cellulose, cetearyl alcohol, alginates, silicon dioxide, aluminum silicates, fumed silica, polysaccharides and their derivatives (e.g., xanthan gum and/or sclerotium gum), polyacrylates (e.g carbomer), acrylates / C10-30 alkyl acrylate crosspolymer, acrylates copolymer, ammonium acryloyldimethyltaurate / VP copolymer and sodium acryloyldimethyltaurate/VP crosspolymer. The amount of thickener in the compositions of the present invention can be from 0.001 wt.% and 5 wt.%, preferably from 0.01 wt. % to about 1 wt. %.

Examples of propellants include volatile hydrocarbons, such as n-butane, propane or isobutane, chlorinated and/or fluorinated hydrocarbons, and mixtures thereof. It is also possible to use, as propellant, carbon dioxide gas, nitrous oxide, dimethyl ether, nitrogen, compressed air, and mixtures thereof. The amount of propellant in the compositions of the present invention can be from 1 wt.% and 25 wt.%.

Additionally, the composition of the present invention can contain other ingredients, such as waterproofing agents, anti-foams, fragrances, pigments, colorants, opacifiers, exfoliants, boosters and other components known in the state of the art for use in topical formulations. The composition of the present disclosure can be prepared by conventional methods well known to those skilled in the art. The method of making the composition may be carried out under normal processing conditions at ambient pressure and with conventional mixing techniques.

The compositions of the present disclosure can be formulated in different ways, such as in the form of creams, ointments, milks, suspensions, powders, oils, lotions, gels, sticks, foams, emulsions (e.g., oil-in-water (O/W), water-in-oil (W/O), water-in-oil-in-water (W/O/W) or oil-in-water-in-oil (O/W/O)), dispersions, sprays and aerosols, and the like. More specific forms include lipsticks, foundations, makeup, loose or press powders, eye blushes, eye shadows, mascaras, nail varnishes, nail lacquers and non-permanent dyeing compositions for the hair, and the like.

The appropriate process for the preparation of each one formulation can readily be determined by those skilled in the art. As an instance, the process for the preparation of an emulsion comprises: (i) firstly, preparing the aqueous phase(s) and the oil phase separately; and (ii) secondly, mixing both phases until obtaining the oil-in-water-emulsion as it is shown in the experimental section.

By way of illustration, the oil-in-water emulsion composition can be prepared following the general process as defined herein below:
(i) preparing an aqueous phase A by mixing the water-soluble components such as preservatives, film-forming agents, chelating agents and humectants, among others with into an aqueous solvent.
(ii) preparing an oil phase B by mixing the sunscreen active agents, the emollient, the emulsifier, and optionally the surfactants, fragrances, and other non-water soluble components;
(iii) optionally, preparing phase C by mixing the preservatives, antioxidants, and additional active ingredients;
(iv) mixing the phases A and B; and
(v) optionally mixing the resulting mixture of step (iv) with phase C.

For the purposes of the present invention, the term "aqueous solvent" refers to a water-based solvent such as water, that is a solvent having water as the major component such as water or a mixture of water with an alkanol such as ethanol.

In an example, the water phase can be present in an amount of greater than 50 wt.% of the composition. The oil phase can be present in an amount less than 50 wt.% weight of the composition. In preferred embodiments, the aqueous phase accounts for from 55 to 97 wt.% and more preferably 57 to 70 wt.% of the composition, and the oil phase accounts for from 3 to 35 wt.% and more preferably 7 to 15 wt.% of the composition.

In addition, the preparation according to the invention can contain one or more active ingredients selected from the group of the compounds alpha-lipoic acid, folic acid, phytoene, D-biotin, coenzyme Q10, alpha-glucosyl rutin, carnitine, carnosine, natural and / or synthetic isoflavonoids, flavonoids, creatine, creatinine, taurine, β-alanine, panthenol, magnolol, honokiol, dihydroxyacetone; 8-hexadecen-1, 16-dicarboxylic acid, glycerylglycose, (2-hydroxyethyl) urea, vitamin E or its derivatives, and / or licochalcone A.

As mentioned above, another aspect of the present invention relates to a method for increasing the photochemical conversion rate of a compound of formula (I) as defined above, such as methoxyphenylethenyl t-butylbenzoate, into a compound of Formula (II) as defined above, such as avobenzone, in a cosmetic composition, the method comprising adding to the composition an effective amount of a phenylethyl ester as defined above.

In an embodiment of the method of the present invention, the phenylethyl ester is phenethyl benzoate. In the present disclosure, it is noted that when discussing the cosmetic composition and the method for increasing the photochemical conversion rate of photochemical precursor of ultraviolet (UV) absorber, the compound of formula (I), or the phenylethyl ester as defined above, each one of the embodiments or features defined for any of the mentioned aspects can be considered applicable to the other aspect, whether or not they are explicitly discussed in the context of that other aspect. Thus, for example, when defining embodiments concerning to the definition of the compound of formula (I) or the compound of formula (II), a pharmaceutically or a cosmetically acceptable salt thereof, a stereoisomer of any of them, or mixtures thereof as defined above in the composition, such embodiments also refer to the definition of the compounds in the method, and *vice versa.*

Throughout the description and claims the word "comprise" and variations of the word, are not intended to exclude other technical features, additives, components, or steps. Furthermore, the word "comprise" encompasses the case of "consisting of". The following examples and drawings are provided by way of illustration, and they are not intended to be limiting of the present disclosure. Furthermore, the present disclosure covers all possible combinations of particular and preferred embodiments described herein.

### Examples

### Instruments and software

- Substrate: HD6 Poly(methyl methacrylate) (PMMA) plates (Helioscreen);
- UV transmittance analizer: UV 2000-S (Labsphere, Inc.);
- Sun simulator: Suntest CPS+ (Atlas Material Testing Technology);
- Water cooling unit for temperature control of the sample: ECO RE 630 S (Lauda);
- Suntest CPS+ lamp: Xenon Lamp NXE 1500B (Atlas Material Testing Technology);
- Software for diffuse transmittance measuring: AS-02784-000 for UV 2000-S (Labpshere, Inc.).

### EXAMPLE 1 - Preparation of methoxyphenylethenyl t-butylbenzoate solutions in different emollients

Samples were prepared by dissolving a 2 % (w/w) of methoxyphenylethenyl t-butylbenzoate in the emollient shown in Table 1 below.

**Table 1**

| **Sample** | **Emollient** |
|---|---|
| Ex. 1 | phenethyl benzoate (PEB) |
| Comp. Ex. 1 | C12-15 alkyl benzoate (C12-15 AB) |
| Comp. Ex. 2 | dibutyl adipate (DBA) |
| Comp. Ex. 3 | diisopropyl adipate (DIPA) |
| Comp. Ex. 4 | dicaprylyl carbonate (DCC) |
| Comp. Ex. 5 | cocoglycerides (CCG) |
| Comp. Ex. 6 | caprylic/capric triglycerides (CCT) |

The solutions are prepared at room temperature (i.e., at about 20-25 °C) with agitation until complete dissolution.

In order to evaluate the effect of different emollients on the photoactivation of methoxyphenylethenyl t-butylbenzoate, an in vitro study with the formulations of Table 1 above was carried out.

The formulations were applied on poly(methyl methacrylate) (PMMA) plates. For each one of the samples above (Example 1 and Comparative Examples 1-6), a plate was prepared by applying 0.68 mg/cm² of the sample on a substrate. The samples were placed in small and uniform drops using a micropipette. Thereafter, they were extended along the substrate: first in small circles for 30 seconds from left to right, and then making horizontal lines for 20 to 30 seconds. The plates were left protected from light for 30-45 min. Three replicates were prepared for each sample.

First, before irradiation, the diffuse transmittance of each sample in the UV range from 250 and 450 nm was measured using a transmittance analyzer (Labsphere UV-2000S). Each sample is measured in 5 different points of the substrate.

Then, the samples were irradiated in a solar simulator (Atlas Suntest CPS+ lamp) for 2 hours in the full UV range (between 300 and 800 nm) and with a potency of 765 W/m². In order to obtain absorbance, SPF, and UVA-PF data, measurements were taken with the transmittance analyzer at the following irradiation times: 1 MED, 2.5 MED, 5 MED, 7.5 MED, and 10 MED (120 minutes of irradiation), wherein 5 MED are 60 minutes of irradiation.

SPF and UVA-PF values were obtained with the software for diffuse transmittance measuring (AS-02784-000).

The behavior of the methoxyphenylethenyl t-butylbenzoate was observed as the irradiation increased.

The results are shown in Tables 2 to 8 below.

**Table 2**

| **PEB + 2% methoxyphenylethenyl t-butylbenzoate** | | | | | | |
|---|---|---|---|---|---|---|
| **MED** | 0 | 1 | 2.5 | 5 | 7.5 | 10 |
| **Abs 360nm** | 0.00 | 0.20 | 0.34 | 0.45 | 0.49 | 0.49 |
| **SPF** | 0.99 | 1.11 | 1.22 | 1.32 | 1.37 | 1.38 |
| **UVA-PF** | 0.92 | 1.34 | 1.79 | 2.22 | 2.37 | 2.38 |

**Table 3**

| **DBA** + **2% methoxyphenylethenyl t-butylbenzoate** | | | | | | |
|---|---|---|---|---|---|---|
| **MED** | 0 | 1 | 2.5 | 5 | 7.5 | 10 |
| **Abs 360nm** | 0.00 | 0.26 | 0.42 | 0.51 | 0.52 | 0.51 |
| **SPF** | 1.02 | 1.23 | 1.39 | 1.50 | 1.52 | 1.51 |
| **UVA-PF** | 0.96 | 1.60 | 2.18 | 2.59 | 2.63 | 2.54 |

**Table 4**

| **DIPA + 2% methoxyphenylethenyl t-butylbenzoate** | | | | | | |
|---|---|---|---|---|---|---|
| **MED** | 0 | 1 | 2.5 | 5 | 7.5 | 10 |
| **Abs 360nm** | 0.00 | 0.25 | 0.44 | 0.55 | 0.54 | 0.54 |
| **SPF** | 0.95 | 1.12 | 1.28 | 1.41 | 1.44 | 1.44 |
| **UVA-PF** | 0.91 | 1.48 | 2.09 | 2.62 | 2.58 | 2.57 |

**Table 5**

| **C12-15 AB + 2% methoxyphenylethenyl t-butylbenzoate** | | | | | | |
|---|---|---|---|---|---|---|
| **MED** | 0 | 1 | 2.5 | 5 | 7.5 | 10 |
| **Abs 360nm** | 0.00 | 0.15 | 0.18 | 0.19 | 0.14 | 0.08 |
| **SPF** | 0.96 | 1.05 | 1.07 | 1.09 | 1.07 | 0.99 |
| **UVA-PF** | 0.92 | 1.24 | 1.33 | 1.38 | 1.25 | 1.06 |

**Table 6**

| **DCC + 2% methoxyphenylethenyl t-butylbenzoate** | | | | | | |
|---|---|---|---|---|---|---|
| **MED** | 0 | 1 | 2.5 | 5 | 7.5 | 10 |
| **Abs 360nm** | 0.00 | 0.11 | 0.14 | 0.12 | 0.10 | 0.05 |
| **SPF** | 0.94 | 0.99 | 1.02 | 1.01 | 1.00 | 0.95 |
| **UVA-PF** | 0.90 | 1.13 | 1.19 | 1.16 | 1.11 | 1.00 |

**Table 7**

| **CCG + 2% methoxyphenylethenyl t-butylbenzoate** | | | | | | |
|---|---|---|---|---|---|---|
| **MED** | 0 | 1 | 2.5 | 5 | 7.5 | 10 |
| **Abs 360nm** | 0.00 | 0.19 | 0.26 | 0.25 | 0.20 | 0.16 |
| **SPF** | 1.04 | 1.14 | 1.19 | 1.18 | 1.14 | 1.08 |
| **UVA-PF** | 0.96 | 1.37 | 1.58 | 1.55 | 1.41 | 1.27 |

**Table 8**

| **CCT + 2% methoxyphenylethenyl t-butylbenzoate** | | | | | | |
|---|---|---|---|---|---|---|
| **MED** | 0 | 1 | 2.5 | 5 | 7.5 | 10 |
| **Abs 360nm** | 0.00 | 0.17 | 0.20 | 0.18 | 0.15 | 0.12 |
| **SPF** | 0.99 | 1.09 | 1.10 | 1.08 | 1.05 | 1.03 |
| **UVA-PF** | 0.93 | 1.27 | 1.36 | 1.31 | 1.22 | 1.15 |

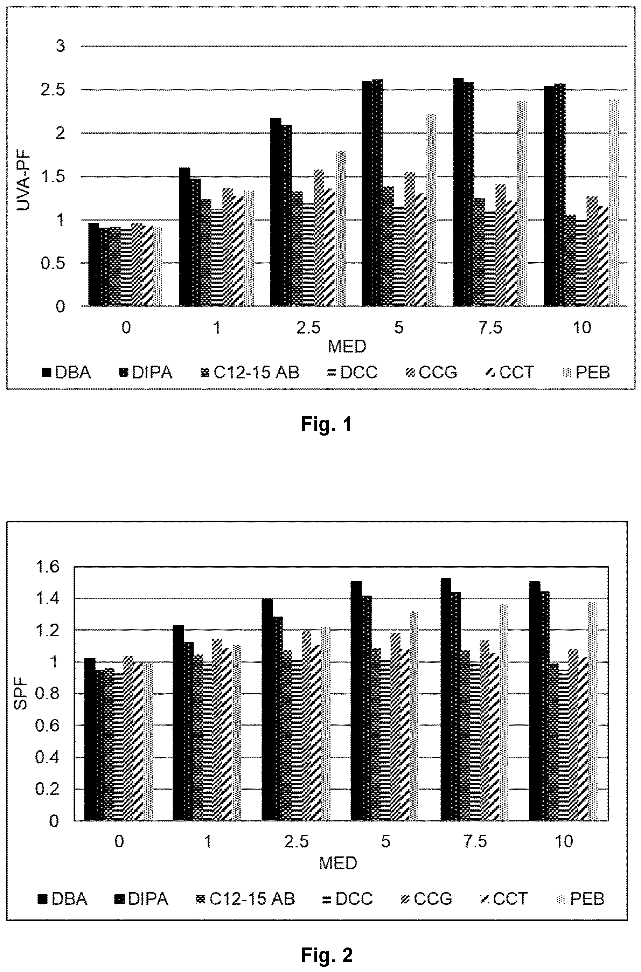

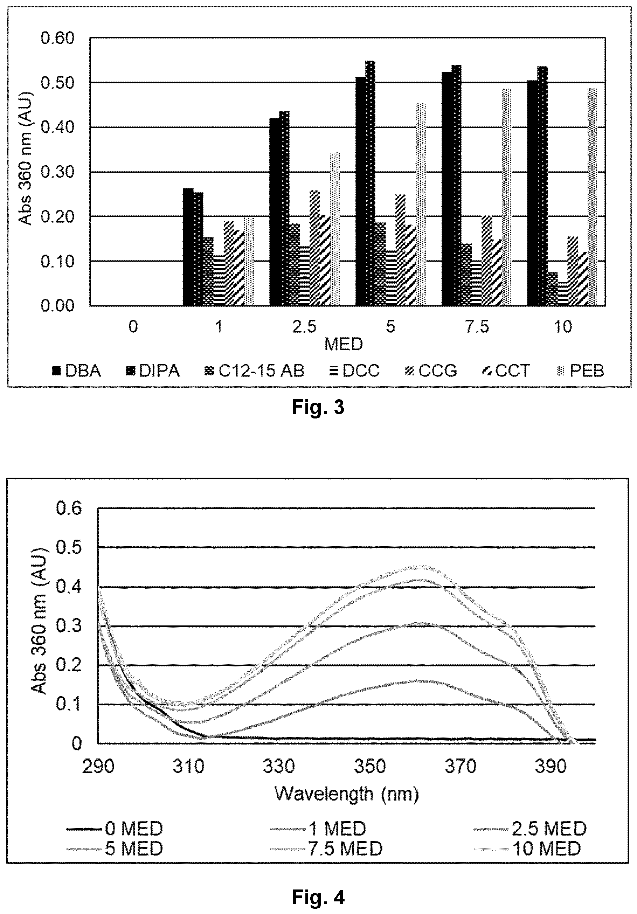

As can be seen in Fig. 1, the results show that the compositions of methoxyphenylethenyl t-butylbenzoate with both the two alkyl adipates (dibutyl adipate and diisopropyl adipate) and phenetyl benzoate provide a similar UVA-PF, which is higher than expected compared to the one obtained with the other emollients. Similarly, as shown in Fig. 3, absorbance at 360nm (within UVA range) is higher for the two alkyl adipates and phenetyl benzoate, than for the rest of emollients. As observed in Fig. 2, SPF values are also higher. This effect could be due to the slight increase of values around 300-320 nm (UVB range) that can be observed in Fig. 4.

### EXAMPLE 2 - Activation rate

Absorbance at 360 nm data obtained for Example 1, Comparative Example 1, and Comparative Example. 2 was used to calculate activation rate.

The activation rate is the time that takes methoxyphenylethenyl t-butylbenzoate to convert into avobenzone. This rate is the slope of the linear trendline obtained by using the first three values that correspond to 0 MED, 1 MED, and 2.5 MED. These results determine how fast the conversion takes place: the higher the slope value, the faster the conversion.

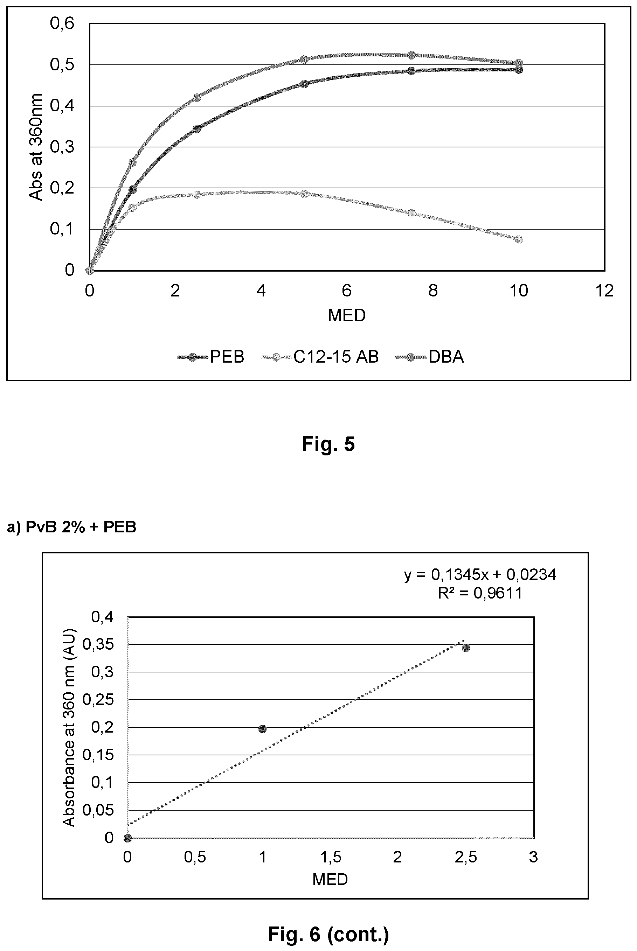

As observed in Fig. 5, Example 1 and Comparative Example 2 have a similar high activation rate compared to the slow activation when using Comparative Example 1. The activation rate values obtained are shown in Table 9.

**Table 9**

| **Sample** | **Activation rate value** |
|---|---|
| Ex. 1 | 0.13 |
| Comp. Ex. 1 | 0.06 |
| Comp. Ex. 2 | 0.16 |

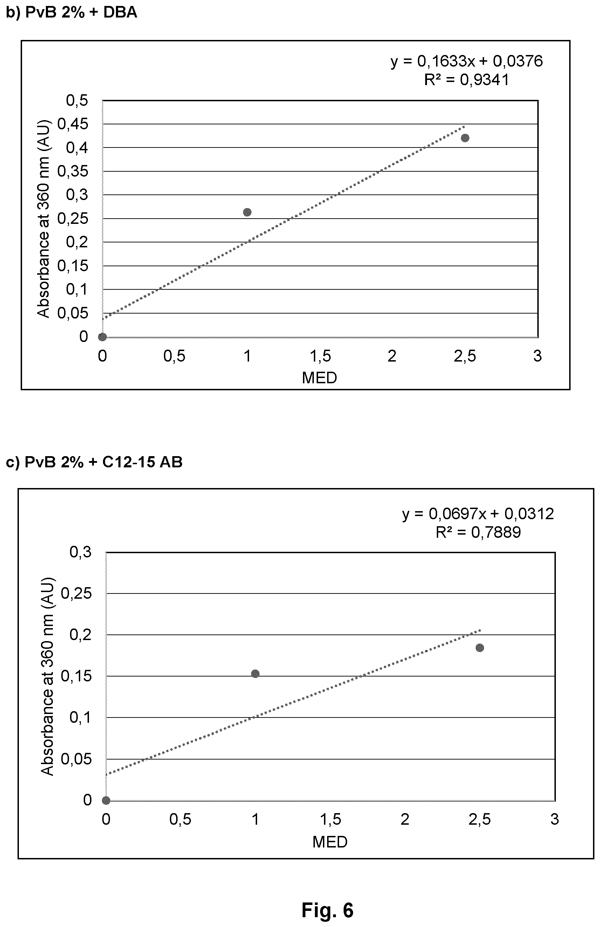

As can be seen in Figs. 5 and 6, the results show higher slope values for Example 1 and Comparative Example 2 compared to Comparative Example 1, what means that the activation rate of methoxyphenylethenyl t-butylbenzoate is significantly increased both with dibutyl adipate and phenethyl benzoate with respect to C12-15 alkyl benzoate. The effect of phenethyl benzoate in the activation rate of methoxyphenylethenyl t-butylbenzoate could not be predicted in view of the effect of dibutyl adipate on the activation rate of methoxyphenylethenyl t-butylbenzoate, since both emollients are structurally and chemically very different.

### EXAMPLE 3 - Preparation of methoxyphenylethenyl t-butylbenzoate in oil phases

Several oil phases containing the components shown in Table 10 below were prepared.

**Table 10**

| **INCI** | **A Comp. Ex. 7** | **B Comp. Ex. 8** | **C Comp. Ex. 9** | **D Ex. 2** |
|---|---|---|---|---|
| Methoxyphenylethenyl t-butylbenzoate | - | 5.6% | - | 5.4% |
| Homosalate | 26.5% | 25.0% | 14.3% | 13.5% |
| Bis-Ethylhexyloxyphenol Methoxyphenyl Triazine | 11.8% | 11.1% | 11.4% | 10.8% |
| Diethylamino hydroxybenzoyl hexyl benzoate | 5.9% | 5.6% | 5.7% | 5.4% |
| Ethylhexyl Triazone | 11.8% | 11.1% | 11.4% | 10.8% |
| Dibutyl Adipate | 44.0% | 41.6% | 42.9% | 40.6% |
| Phenethyl Benzoate | - | - | 14.3% | 13.5% |
| Total | 100.0% | 100.0% | 100.0% | 100.0% |

As an instance, an emulsion containing from 30 wt.% to 40 wt.% of the oil phase above can be prepared.

As an example, the final amounts of the different components in the final composition, when present, can be:
- methoxyphenylethenyl t-butylbenzoate: 2 wt.%;
- homosalate: 5 wt.%;
- bis-ethylhexyloxyphenol methoxyphenyl triazine: 4 wt.%;
- diethylamino hydroxybenzoyl hexyl benzoate: 2 wt.%;
- ethylhexyl triazone: 4 wt.%;
- dibutyl adipate: 15 wt.%;
- phenethyl benzoate: 5 wt.%.

When a mixture of emollients (that is, of phenethyl benzoate and dibutyl adipate) was used previously to their admixture with the rest of components, they were mixed in a bath at a temperature of 70-75 °C.

Methoxyphenylethenyl t-butylbenzoate and the sunscreen active agents were then added to the emollient or mixture of emollients one at a time. Each sunscreen active agent is left stirring for 10 minutes or until complete solubilization at the mentioned temperature.

Finally, the mixture was cold down under stirring to room temperature.

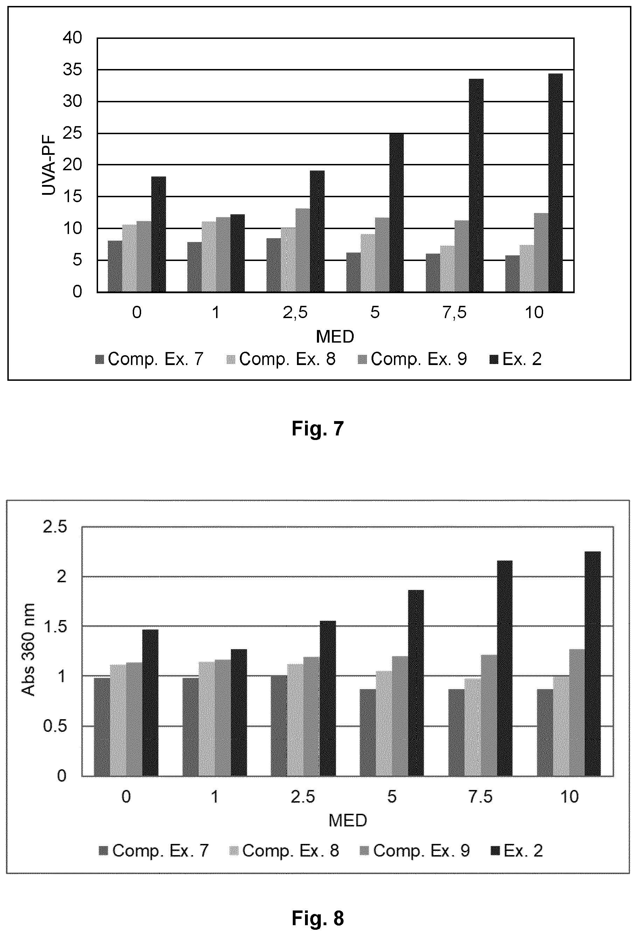

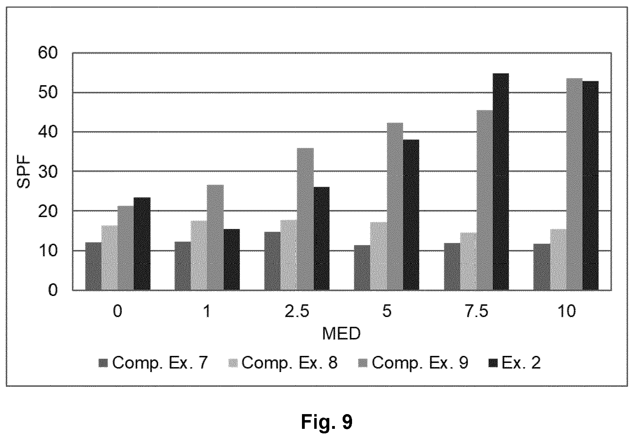

The results show that the oil phase D containing both methoxyphenylethenyl t-butylbenzoate and phenethyl benzoate (Example 2) provides surprisingly high UVA-PF protection (Fig. 7), absorbance at 360 nm (Fig. 8), and SPF protection (Fig. 9) compared with any of the other oil phases A (Comparative Example 7), B (A + methoxyphenylethenyl t-butylbenzoate; Comparative Example 8), and C (A + phenethyl benzoate; Comparative Example 9).

Even though, as has been seen in Example 1, in the absence of sunscreen active agents, dibutyl adipate has an enhancing effect on the conversion of methoxyphenylethenyl t-butylbenzoate to avobenzone, in the presence of other sunscreen active agents (particularly of homosalate, bis-ethylhexyloxyphenol methoxyphenyl triazine, diethylamino hydroxybenzoyl hexyl benzoate, and ethylhexyl triazone), the addition of phenethyl benzoate without methoxyphenylethenyl t-butylbenzoate does not lead a significant increase of the UVA-PF, but just helps to maintain the UVA-PF (possibly due to the solubilization effect of phenethyl benzoate on at least some of the sunscreen active agents).

Conversely, unexpectedly, when phenethyl benzoate is added to the composition comprising methoxyphenylethenyl t-butylbenzoate (oil phase D) it can be observed a significantly high increase of UVA-PF protection, even though phenethyl benzoate is added in an amount that is a third of the amount of dibutyl adipate.

This was unexpected since, as commented above, in the absence of the sunscreen active agents, the UVA-PF protection and absorbances of the combination of methoxyphenylethenyl t-butylbenzoate with dibutyl adipate were similar to, or slightly better than, the ones provided by the combination of methoxyphenylethenyl t-butylbenzoate with phenethyl benzoate. Thus, without wishing to be bound by theory, the results seem to indicate that the presence of the other sunscreen active agents interferes with the action of the dibutyl adipate on one or more components of the composition in contrast to phenethyl benzoate.

It is worth mentioning that avobenzone is an UVA filter, thus, providing a UVA-PF protection. Therefore, this is in concordance with the results for the SPF, showing no substantial improvement with the presence of methoxyphenylethenyl t-butylbenzoate in the oil phase.

### EXAMPLE 4 - Sunscreen formulation

The formulation of Table 11 was prepared. The ingredients of Phase B, Phase C, and Phase D were prepared in separate suitable containers.

**Table 11**

| **PHASE** | **INCI** | **%w/w** |
|---|---|---|
| A | Water (aqua) | qsp 100 |
| A | Tetrasodium EDTA | 0.20% |
| A | Glycerin | 4.00% |
| A | Xanthan Gum (and) Lecithin (and) Sclerotium Gum (and) Pullulan | 1.00% |
| A | Preservative | qs |
| B | Dibutyl Adipate | 15.00% |
| B | Phenethyl Benzoate | 5.00% |
| B | Bis-Ethylhexyloxyphenol Methoxyphenyl Triazine | 2.00% |
| B | Ethylhexyl Triazone | 2.50% |
| B | Methoxyphenylethenyl *t*-butylbenzoate | 2.00% |
| B | Ethylcellulose (and) Diisopropyl Adipate | 0.50% |
| B | Poly C10-30 Alkyl Acrylate | 2.00% |
| B | O/W emulsifier | 0.50% |
| B | Lauryl Glucoside (and) Myristyl Glucoside (and) Polyglyceryl-6 Laurate | 2.00% |
| B | Antioxidant | 0.50% |
| C | Methylene Bis-Benzotriazolyl Tetramethylbutylphenol (and) Aqua (and) Decyl Glucoside (and) Propylene Glycol (and) Xanthan Gum | 6.00% |
| D | pH adjust | qs |
| Total | | 100.00% |

An aqueous Phase A was prepared by combining the ingredients and heating to a temperature of 70-80 °C until obtaining a homogeneous mixture. An oil Phase B was prepared similarly as in Example 2. Then, the two phases, at the same temperature, where emulsified by adding phase B to phase A using a homogenizer. Then, the mixture was cooled down under stirring to room temperature, Phase C was added, and the pH was adjusted.

### EXAMPLE 5 - Color formulation

The formulation of Table 12 was prepared. The ingredients of Phase B, Phase C and Phase D were prepared in separate suitable containers.

**Table 12**

| **PHASE** | **INCI** | **%w/w** |
|---|---|---|
| A | Water (Aqua) | qsp 100 |
| A | Tetrasodium EDTA | 0.20% |
| A | Glycerin | 6.00% |
| B | O/W Emulsifier | 5.00% |
| B | C10-18 Trglycerides | 4.00% |
| B | Prunus Amygdalus Dulcis Oil | 3.00% |
| B | C12-15 Alkyl Benzoate (And) Stearalkonium Hectorite (And) Propylene Carbonate | 3.00% |
| B | Phenethyl Benzoate | 2.00% |
| B | Dibutyl Adipate | 10.00% |
| B | Methoxyphenylethenyl T-Butylbenzoate | 2.00% |
| B | Coco-Caprylate/Caprate | 3.00% |
| B | Isostearyl Isostearate | 3.00% |
| B | Iron Oxides CI 77891 (And) Silica | 6.00% |
| B | Iron Oxides CI 77492 (And) Silica | 0.60% |
| B | Iron Oxides CI 77491 (And) Silica | 0.30% |
| B | Iron Oxides CI 77499 (And) Silica | 0.06% |
| B | Antioxidant | 0.50% |
| C | Preservative | 1.00% |
| D | Ph Adjust | qs |
| Total | | 100.00% |

An aqueous Phase A was prepared by combining the ingredients and heating to a temperature of 70-80 °C until obtaining a homogeneous mixture. An oil Phase B was prepared by combining the ingredients under rapid mixing (rotor/stator 1500 rpm) and heating to a temperature of 70-80 °C. Then, the two phases, at the same temperature, where emulsified by adding phase B to phase A using a homogenizer. Then, the mixture was cooled down under stirring to room temperature, Phase C was added, and the pH was adjusted.

### Citation List

1. WO2006/100225A2
2. Annex VI to Regulation (EC) No 1223/2009 (update 22/09/2021)

## Claims

1. A composition comprising:
- an effective amount of a compound of formula (I), a pharmaceutically or a cosmetically acceptable salt thereof, or a stereoisomer of any of them or mixtures thereof, 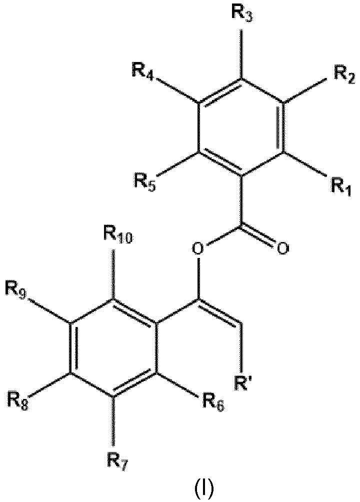 wherein: R' is selected from the group consisting of H, (C₁-C₆)-alkyl, and (C₃-C₆)-cycloalkyl; R₁, R₂, R₄, R₅, R₆, R₇, R₉, and R₁₀ are a radical independently selected from the group consisting of H, hydroxy, amino, (C₁-C₆)-alkyl, (C₁-C₆)-alkoxy, (C₁-C₆)-alkylamino, and (C₁-C₆)-dialkylamino; and R₃ and R₈ are independently selected from the group consisting of (C₁-C₆)-alkyl, (C₁-C₆)-alkoxy, hydroxy, amino, (C₁-C₆)-alkylamino, and (C₁-C₆)-dialkylamino; and
- an effective amount of a phenylethyl ester selected from the group consisting of phenethyl benzoate, 2-phenylethyl toluate, di-2-phenylethyl phthalate, and 1-phenylethyl benzoate;
together with one or more pharmaceutically or cosmetically acceptable excipients or carriers.

2. The composition according to claim 1, wherein R' is H.

3. The composition according to claims 1 or 2, wherein R₁, R₂, R₄, R₅, R₆, R₇, R₉, and R₁₀ are a radical independently selected from the group consisting of H, hydroxy, amino, and methyl.

4. The composition according to any one of claims 1 to 3, wherein R₂, R₄, R₇, and R₉ are H.

5. The composition according to any one of claims 3 to 4, wherein R', R₁, R₂, R₄, R₅, R₆, R₇, R₉, and R₁₀ are H; R₃ is tert-butyl and R₈ is methoxyl.

6. The composition according to any one of claims 1 to 5, wherein the phenylethyl ester is phenethyl benzoate.

7. The composition according to any one of claims 1 to 6, wherein the compound of formula (I) is in an amount from 0.1 wt.% to 6 wt.% and the phenylethyl ester is in an amount from 0.1 wt.% to 40 wt.%, relative to the total weight of the composition, being the sum of all components 100%.

8. The composition according to any one of claims 1 to 7, further comprising a UV sunscreen active agent.

9. The composition according to any one of claims 1 to 8, wherein the UV sunscreen active agent is in an amount from 0.1 wt.% to 50 wt.%, being the sum of all components 100%.

10. The composition according to any one of claims 1 to 9, further comprising an emollient other than the phenylethyl ester.

11. The composition according to claims 10, wherein the emollient other than the phenylethyl ester is in an amount 0.5 wt.% to 35 wt.%, relative to the total weight of the composition, being the sum of all components 100%.

12. The composition according to claims 10 or 11, wherein the emollient other than the phenylethyl ester is a dialkyl adipate.

13. The composition according to claim 12, wherein the UV sunscreen agent comprises an UVB sunscreen active agent, an UVA sunscreen active agent, and a UVA/UVB sunscreen active agent, the compound of formula (I) is methoxyphenylethenyl t-butylbenzoate, and
- the methoxyphenylethenyl t-butylbenzoate is in an amount from 0.1 wt.% to 6 wt.%;
- the phenylethyl ester is in an amount from 0.1 wt.% to 40 wt.%;
- the UVB absorber is in an amount from 0.1 wt.% to 25 wt.%;
- the UVA absorber is in an amount from 0.1 wt.% to 15 wt.%;
- the UVA/UVB absorber is in an amount from 0.1 wt.% to 15 wt.%; and
- the dialkyl adipate is in an amount from 0.5 wt.% to 35 wt.%,
wherein the weight percentages are relative to the total weight of the composition, being the sum of components up to 100%.

14. Composition according to claims 1 to 13 for use to provide protection for the skin or hair against the sun's rays or other sources of UV radiation.

15. Use of a phenylethyl ester selected from the group consisting of phenethyl benzoate, 2-phenylethyl toluate, di-2-phenylethyl phthalate, and 1-phenylethyl benzoate for increasing the photochemical conversion rate of a compound of formula (I) as defined in claims 1 to 5 into a compound of Formula (II), a pharmaceutically or a cosmetically acceptable salt thereof, or a stereoisomer of any of them or mixtures thereof, 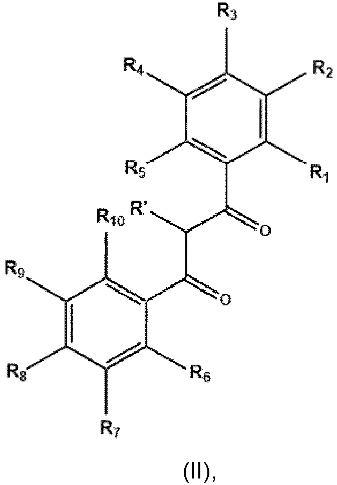 wherein: R' is selected from the group consisting of H, (C₁-C₆)-alkyl, and (C₃-C₆)-cycloalkyl; R₁, R₂, R₄, R₅, R₆, R₇, R₉, and R₁₀ are a radical independently selected from the group consisting of H, hydroxy, amino, (C₁-C₆)-alkyl, (C₁-C₆)-alkoxy, (C₁-C₆)-alkylamino, and (C₁-C₆)-dialkylamino; and R₃ and R₈ are independently selected from the group consisting of (C₁-C₆)-alkyl, (C₁-C₆)-alkoxy, hydroxy, amino, (C₁-C₆)-alkylamino, and (C₁-C₆)-dialkylamino, in a cosmetic composition.

## Patentansprüche

1. Eine Zusammensetzung umfassend:
- eine wirksame Menge einer Verbindung der Formel (I), eines pharmazeutisch oder kosmetisch akzeptablen Salzes davon oder eines Stereoisomers eines davon oder einer Mischung davon, 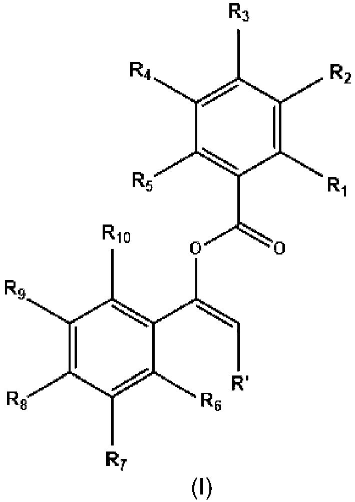 wobei: R' ausgewählt ist aus der Gruppe bestehend aus H, (C₁-C₆)-Alkyl und (C₃-C₆)-Cycloalkyl; R_{1,} R₂, R₄, R_{5,} R_{6,} R₇, R₉ und R₁₀ ein Rest sind, der unabhängig ausgewählt ist aus der Gruppe bestehend aus H, Hydroxy, Amino, (C₁-C₆)-Alkyl, (C₁-C₆)-Alkoxy, (C₁-C₆)-Alkylamino und (C₁-C₆)-Dialkylamino; und R₃ und R₈ unabhängig voneinander ausgewählt sind aus der Gruppe bestehend aus (C₁-C₆)-Alkyl, (C₁-C₆)-Alkoxy, Hydroxy, Amino, (C₁-C₆)-Alkylamino und (C₁-C₆)-Dialkylamino; und
- eine wirksame Menge eines Phenylethylesters, der ausgewählt ist aus der Gruppe bestehend aus Phenethylbenzoat, 2-Phenylethyltoluat, Di-2-phenylethylphthalat und 1-Phenylethylbenzoat;
zusammen mit einem oder mehreren pharmazeutisch oder kosmetisch akzeptablen Hilfsstoffen oder Trägersubstanzen.

2. Die Zusammensetzung nach Anspruch 1, wobei R' = H ist.

3. Die Zusammensetzung nach Anspruch 1 oder 2, wobei R₁, R₂, R₄, R₅, R₆, R₇, R₉ und R₁₀ ein Rest sind, der unabhängig ausgewählt ist aus der Gruppe bestehend aus H, Hydroxy, Amino und Methyl.

4. Die Zusammensetzung nach einem der Ansprüche 1 bis 3, wobei R₂, R₄, R₇ und R₉ = H sind.

5. Die Zusammensetzung nach einem der Ansprüche 3 bis 4, wobei R', R₁, R₂, R₄, R₅, R₆, R₇, R₉ und R₁₀ = H sind; R₃ = tert-Butyl ist und R₈ = Methoxyl ist.

6. Die Zusammensetzung nach einem der Ansprüche 1 bis 5, wobei der Phenylethylester Phenethylbenzoat ist.

7. Die Zusammensetzung nach einem der Ansprüche 1 bis 6, wobei die Verbindung der Formel (I) in einer Menge von 0,1 Gew.- % bis 6 Gew.- % und der Phenylethylester in einer Menge von 0,1 Gew.- % bis 40 Gew.- %, bezogen auf das Gesamtgewicht der Zusammensetzung, vorliegt, wobei die Summe aller Komponenten 100 % beträgt.

8. Die Zusammensetzung nach einem der Ansprüche 1 bis 7, weiterhin umfassend einen UV-Sonnenschutzwirkstoff.

9. Die Zusammensetzung nach einem der Ansprüche 1 bis 8, wobei der UV-Sonnenschutzwirkstoff in einer Menge von 0,1 Gew.- % bis 50 Gew.- % vorliegt, wobei die Summe aller Komponenten 100 % beträgt.

10. Die Zusammensetzung nach einem der Ansprüche 1 bis 9, weiterhin umfassend ein anderes Emolliens als den Phenylethylester.

11. Die Zusammensetzung nach Anspruch 10, wobei das andere Emolliens als der Phenylethylester in einer Menge von 0,5 Gew.- % bis 35 Gew.- %, bezogen auf das Gesamtgewicht der Zusammensetzung, vorliegt, wobei die Summe aller Komponenten 100 % beträgt.

12. Die Zusammensetzung nach Anspruch 10 oder 11, wobei das andere Emolliens als der Phenylethylester ein Dialkyladipat ist.

13. Die Zusammensetzung nach Anspruch 12, wobei das UV-Sonnenschutzmittel einen UVB-Sonnenschutzwirkstoff, einen UVA-Sonnenschutzwirkstoff und einen UVA/UVB-Sonnenschutzwirkstoff umfasst, die Verbindung der Formel (I) Methoxyphenylethenyl-t-butylbenzoat ist, und
- das Methoxyphenylethenyl-t-butylbenzoat in einer Menge von 0,1 Gew.- % bis 6 Gew.- % vorliegt;
- der Phenylethylester in einer Menge von 0,1 Gew.- % bis 40 Gew.- % vorliegt;
- der UVB-Absorber in einer Menge von 0,1 Gew.- % bis 25 Gew.- % vorliegt;
- der UVA-Absorber in einer Menge von 0,1 Gew.- % bis 15 Gew.- % vorliegt;
- der UVA/UVB-Absorber in einer Menge von 0,1 Gew.- % bis 15 Gew.- % vorliegt; und
- das Dialkyladipat in einer Menge von 0,5 Gew.- % bis 35 Gew.- % vorliegt,
wobei die Gewichtsprozente auf das Gesamtgewicht der Zusammensetzung bezogen sind, wobei die Summe der Komponenten bis zu 100 % beträgt.

14. Zusammensetzung nach den Ansprüchen 1 bis 13 zur Verwendung, um Schutz für die Haut oder das Haar vor Sonnenstrahlen oder anderen UV-Strahlungsquellen bereitzustellen.

15. Verwendung eines Phenylethylesters, der ausgewählt ist aus der Gruppe bestehend aus Phenethylbenzoat, 2-Phenylethyltoluat, Di-2-phenylethylphthalat und 1-Phenylethylbenzoat, zur Erhöhung der photochemischen Umwandlungsrate einer Verbindung der Formel (I), wie in den Ansprüchen 1 bis 5 definiert, in eine Verbindung der Formel (II), ein pharmazeutisch oder kosmetisch akzeptables Salz davon, oder ein Stereoisomer eines davon oder Mischungen davon, 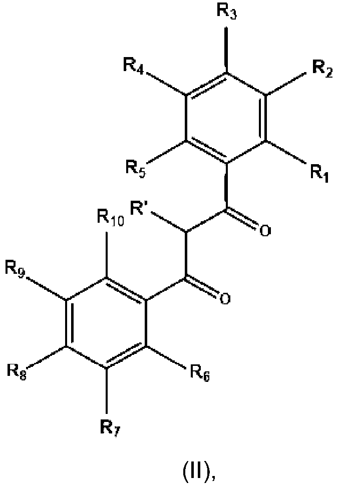 wobei: R' ausgewählt ist aus der Gruppe bestehend aus H, (C₁-C₆)-Alkyl und (C₃-C₆)-Cycloalkyl; R_{1,} R₂, R₄, R_{5,} R_{6,} R₇, R₉ und R₁₀ ein Rest sind, der unabhängig ausgewählt ist aus der Gruppe bestehend aus H, Hydroxy, Amino, (C₁-C₆)-Alkyl, (C₁-C₆)-Alkoxy, (C₁-C₆)-Alkylamino und (C₁-C₆)-Dialkylamino; und R₃ und R₈ unabhängig voneinander ausgewählt sind aus der Gruppe bestehend aus (C₁-C₆)-Alkyl, (C₁-C₆)-Alkoxy, Hydroxy, Amino, (C₁-C₆)-Alkylamino und (C₁-C₆)-Dialkylamino, in einer kosmetischen Zusammensetzung.

## Revendications

1. Une composition comprenant :
- une quantité efficace d'un composé de formule (I), d'un sel pharmaceutiquement ou cosmétiquement acceptable de celui-ci, ou d'un stéréoisomère de l'un d'entre eux ou de mélanges de ceux-ci, 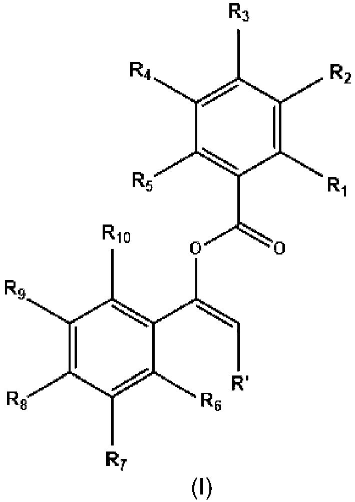 dans laquelle : R' est choisi dans le groupe constitué par H, alkyle en (C₁-C₆) et cycloalkyle en (C₃-C₆) ; R_{1,} R₂, R₄, R_{5,} R_{6,} R₇, R₉ et R₁₀ sont un radical choisi indépendamment dans le groupe constitué par H, hydroxy, amino, alkyle en (C₁-C₆), alcoxy en (C₁-C₆), alkylamino en (C₁-C₆) et dialkylamino en (C₁-C₆) ; et R₃ et R₈ sont choisis indépendamment dans le groupe constitué par alkyle en (C₁-C₆), alcoxy en (C₁-C₆), hydroxy, amino, alkylamino en (C₁-C₆) et dialkylamino en (C₁-C₆) ; et
- une quantité efficace d'un ester phényléthylique choisi dans le groupe constitué par le benzoate de phénéthyle, le toluate de 2-phényléthyle, le phtalate de di-2-phényléthyle et le benzoate de 1-phényléthyle ;
avec un ou plusieurs excipients ou véhicules pharmaceutiquement ou cosmétiquement acceptables.

2. La composition selon la revendication 1, dans laquelle R' est H.

3. La composition selon la revendication 1 ou 2, dans laquelle R₁, R₂, R₄, R₅, R₆, R₇, R₉ et R₁₀ sont un radical choisi indépendamment dans le groupe constitué par H, hydroxy, amino et méthyle.

4. La composition selon l'une quelconque des revendications 1 à 3, dans laquelle R₂, R₄, R₇ et R₉ sont H.

5. La composition selon l'une quelconque des revendications 3 à 4, dans laquelle R', R₁, R₂, R₄, R₅, R₆, R₇, R₉ et R₁₀ sont H ; R₃ est le tert-butyle et R₈ est le méthoxyle.

6. La composition selon l'une quelconque des revendications 1 à 5, dans laquelle l'ester phényléthylique est le benzoate de phénéthyle.

7. La composition selon l'une quelconque des revendications 1 à 6, dans laquelle le composé de formule (I) est en une quantité de 0,1 % en poids à 6 % en poids et l'ester phényléthylique est en une quantité de 0,1 % en poids à 40 % en poids, par rapport au poids total de la composition, étant la somme de tous les composants 100 %.

8. La composition selon l'une quelconque des revendications 1 à 7, comprenant en outre un agent actif de protection solaire contre les UV.

9. La composition selon l'une quelconque des revendications 1 à 8, dans laquelle l'agent actif de protection solaire contre les UV est en une quantité de 0,1 % en poids à 50 % en poids, étant la somme de tous les composants 100 %.

10. La composition selon l'une quelconque des revendications 1 à 9, comprenant en outre un émollient autre que l'ester phényléthylique.

11. La composition selon la revendication 10, dans laquelle l'émollient autre que l'ester phényléthylique est en une quantité de 0,5 % en poids à 35 % en poids, par rapport au poids total de la composition, étant la somme de tous les composants 100 %.

12. La composition selon la revendication 10 ou 11, dans laquelle l'émollient autre que l'ester phényléthylique est un adipate de dialkyle.

13. La composition selon la revendication 12, dans laquelle l'agent de protection solaire contre les UV comprend un agent actif de protection solaire contre les UVB, un agent actif de protection solaire contre les UVA et un agent actif de protection solaire contre les UVA/UVB, le composé de formule (I) est le t-butylbenzoate de méthoxyphényléthényle, et
- le t-butylbenzoate de méthoxyphényléthényle est en une quantité de 0,1 % en poids à 6 % en poids ;
- l'ester phényléthylique est en une quantité de 0,1 % en poids à 40 % en poids ;
- l'absorbeur d'UVB est en une quantité de 0,1 % en poids à 25 % en poids ;
- l'absorbeur d'UVA est en une quantité de 0,1 % en poids à 15 % en poids ;
- l'absorbeur d'UVA/UVB est en une quantité de 0,1 % en poids à 15 % en poids ; et
- l'adipate de dialkyle est en une quantité de 0,5 % en poids à 35 % en poids,
dans lequel les pourcentages en poids sont par rapport au poids total de la composition, étant la somme des composants jusqu'à 100 %.

14. Composition selon les revendications 1 à 13 pour une utilisation pour fournir une protection pour la peau ou les cheveux contre les rayons du soleil ou d'autres sources de rayonnement UV.

15. Utilisation d'un ester phényléthylique choisi dans le groupe constitué par le benzoate de phénéthyle, le toluate de 2-phényléthyle, le phtalate de di-2-phényléthyle et le benzoate de 1-phényléthyle pour augmenter le taux de conversion photochimique d'un composé de formule (I) tel que défini dans les revendications 1 à 5 en un composé de formule (II), un sel pharmaceutiquement ou cosmétiquement acceptable de celui-ci, ou un stéréoisomère de l'un d'entre eux ou des mélanges de ceux-ci, 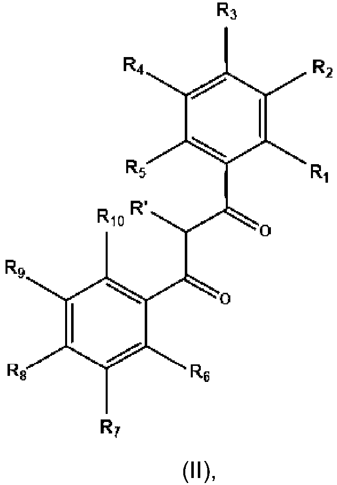 dans laquelle : R' est choisi dans le groupe constitué par H, alkyle en (C₁-C₆) et cycloalkyle en (C₃-C₆) ; R₁, R₂, R₄, R₅, R₆, R₇, R₉ et R₁₀ sont un radical choisi indépendamment dans le groupe constitué par H, hydroxy, amino, alkyle en (C₁-C₆), alcoxy en (C₁-C₆), alkylamino en (C₁-C₆) et dialkylamino en (C₁-C₆) ; et R₃ et R₈ sont choisis indépendamment dans le groupe constitué par alkyle en (C₁-C₆), alcoxy en (C₁-C₆), hydroxy, amino, alkylamino en (C₁-C₆) et dialkylamino en (C₁-C₆), dans une composition cosmétique.
